Europäisches Patentamt

European Patent Office

Office européen des brevets

(19)

(11) **EP 1 159 244 B1**

(12) **EUROPÄISCHE PATENTSCHRIFT**

(45) Veröffentlichungstag und Bekanntmachung des Hinweises auf die Patenterteilung:
**29.10.2003 Patentblatt 2003/44**

(21) Anmeldenummer: **00916877.4**

(22) Anmeldetag: **28.02.2000**

(51) Int Cl.[7]: **C07C 45/35**, C07C 47/22, C07C 57/04

(86) Internationale Anmeldenummer:
**PCT/EP00/01634**

(87) Internationale Veröffentlichungsnummer:
**WO 00/053556 (14.09.2000 Gazette 2000/37)**

(54) **VERFAHREN DER KATALYTISCHEN GASPHASENOXIDATION VON PROPEN ZU ACROLEIN**

METHOD FOR THE CATALYTIC GAS PHASE OXIDATION OF PROPENE INTO ACROLEIN

PROCEDE D'OXYDATION CATALYTIQUE EN PHASE GAZEUSE DE PROPENE POUR FORMER DE L'ALDEHYDE ACRYLIQUE

(84) Benannte Vertragsstaaten:
**BE DE ES FR IT**

(30) Priorität: **10.03.1999 DE 19910506**
**07.10.1999 DE 19948241**

(43) Veröffentlichungstag der Anmeldung:
**05.12.2001 Patentblatt 2001/49**

(73) Patentinhaber: **BASF AKTIENGESELLSCHAFT**
**67056 Ludwigshafen (DE)**

(72) Erfinder:
• **ARNOLD, Heiko**
**D-68159 Mannheim (DE)**
• **UNVERRICHT, Signe**
**D-68169 Mannheim (DE)**
• **HAMMON, Ulrich**
**D-68165 Mannheim (DE)**
• **NEUMANN, Hans-Peter**
**D-67067 Ludwigshafen (DE)**
• **HARTH, Klaus**
**D-67317 Altleiningen (DE)**
• **TENTEN, Andreas**
**D-67487 Maikammer (DE)**

(56) Entgegenhaltungen:
EP-A- 0 015 565    EP-A- 0 253 409
EP-A- 0 293 224    EP-A- 0 450 596
EP-A- 0 900 774    DE-A- 2 513 405

**Beschreibung**

Vorliegende Erfindung betrifft ein Verfahren der katalytischen Gasphasenoxidation von Propen zu Acrolein, bei dem man ein Propen, molekularen Sauerstoff und wenigstens ein Inertgas, das zu wenigstens 20 % seines Volumens aus molekularem Stickstoff besteht, enthaltendes Reaktionsgasausgangsgemisch, das den molekularen Sauerstoff und das Propen in einem molaren Verhältnis $O_2:C_3H_6 \geq 1$ enthält, so über einen Festbettkatalysator, dessen Aktivmasse wenigstens ein Molybdän und/oder Wolfram sowie Wismut, Tellur, Antimon, Zinn und/oder Kupfer enthaltendes Multimetalloxid ist, führt, daß der Propenumsatz bei einmaligem Durchgang $\geq 90$ mol-% und die damit einhergehende Selektivität der Acroleinbildung sowie der Arylsäurenebenproduktbildung zusammengenommen $\geq 90$ mol-% betragen.

Das vorgenannte Verfahren der katalytischen Gasphasenoxidation von Propen zu Acrolein ist allgemein bekannt (vgl. z.B. EP-A 15565, EP-A 700714, DE-C 2830765, DE-C 3338380, JP-A 91/294239, EP-A 807 465, WO 98/24746, EP-B 279374, DE-C 2513405, DE-A 3300044, EP-A 575897 und DE-A 19855913) und insbesondere als erste Oxidationsstufe bei der Herstellung von Acrylsäure durch zweistufige katalytische Gasphasenoxidation von Propen in zwei hintereinandergeschalteten Reaktionsstufen von Bedeutung (vgl. z.B. DE-A 3002829). Acrylsäure ist ein bedeutendes Monomeres, das als solches oder in Form seiner Alkylester zur Erzeugung von z.B. als Klebstoffen geeigneten Polymerisaten Verwendung findet.

Da bei der oben genannten katalytischen Gasphasenoxidation von Propen zu Acrolein normalerweise in geringer Menge Acrylsäurenebenproduktbildung erfolgt und gemäß Vorstehendem Acrylsäure in der Regel das angestrebte natürliche Folgeprodukt von Acrolein ist, wird im Rahmen einer katalytischen Gasphasenoxidation von Propen zu Acrolein normalerweise die molare Summe aus gebildetem Acrolein und als Nebenprodukt gebildeter Acrylsäure als Wertprodukt betrachtet. Diese Betrachtungsweise soll auch in der vorliegenden Patentanmeldung gelten.

Die Zielsetzung einer jeden katalytischen Festbettgasphasenoxidation von Propen zu Acrolein besteht grundsätzlich darin, eine möglichst hohe Raum-Zeit-Ausbeute (RZA) an Wertprodukt zu erzielen (das ist bei einer kontinuierlichen Verfahrensweise die je Stunde und Volumen der verwendeten Katalysatorschüttung in Litern erzeugte Grammmenge an Wertprodukt).

Es besteht deshalb generelles Interesse daran, die Gasphasenoxidation unter einer möglichst hohen Belastung der Katalysatorschüttung mit Propen (darunter wird die Menge an Propen in Normlitern (=N1; das Volumen in Liter, das die entsprechende Propenmenge bei Normalbedingungen, d.h., bei 25°C und 1 bar, einnehmen würde) verstanden, die als Bestandteil des Reaktionsgasgemisches pro Stunde durch einen Liter an Katalysatorschüttung geführt wird) durchzuführen, ohne dabei den bei einmaligem Durchgang des Reaktionsgasausgangsgemisches durch die Katalysatorschüttung erfolgenden Umsatz an Propen sowie die Selektivität der damit einhergehenden Wertproduktbildung nennenswert zu beeinträchtigen.

Die Umsetzung des Vorgenannten wird durch die Tatsache beeinträchtigt, daß die Gasphasenoxidation von Propen zu Acrolein einerseits stark exotherm verläuft und andererseits von einer Vielfalt möglicher Parallel- und Folgereaktionen begleitet wird.

Mit zunehmender Propenbelastung der Katalysatorschüttung muß, bei Verwirklichung der angestrebten Randbedingung eines im wesentlichen gleichbleibenden Propenumsatzes, daher davon ausgegangen werden, daß infolge der erhöhten Wärmeproduktion die Selektivität der Wertproduktbildung abnimmt (vgl. EP-B 450596, Example 1 und 2).

Die konventionellen Verfahren der katalytischen Gasphasenoxidation von Propen zu Acrolein, die dadurch charakterisiert sind, daß als ein Hauptbestandteil des inerten Verdünnungsgases Stickstoff und außerdem ein in einer Reaktionszone befindlicher und längs dieser Reaktionszone homogener, d.h., über die Katalysatorschüttung chemisch einheitlich zusammengesetzter, Festbettkatalysator verwendet und die Temperatur der Reaktionszone auf einem über die Reaktionszone einheitlichen Wert gehalten wird (unter Temperatur einer Reaktionszone wird hier die Temperatur der in der Reaktionszone befindlichen Katalysatorschüttung bei Ausübung des Verfahrens in Abwesenheit einer chemischen Reaktion verstanden; ist diese Temperatur innerhalb der Reaktionszone nicht konstant, so meint der Begriff Temperatur einer Reaktionszone hier den Zahlenmittelwert der Temperatur der Katalysatorschüttung längs der Reaktionszone), beschränken daher den anzuwendenden Wert der Propenbelastung der Katalysatorschüttung auf Werte $\leq 155$ Nl Propen/l Katalysatorschüttung·h (vgl. z.B. EP-A 15565 (maximale Propenlast = 120 Nl Propen/l·h), DE-C 2830765 (maximale Propenlast = 94,5 Nl Propen/l·h), EP-A 807465 (maximale Propenlast = 128 Nl Propen/l·h), EP-B 279374 (maximale Propenlast = 112 Nl Propen/l·h), DE-C 2513405 (maximale Propenlast = 110 Nl Propen/l·h), DE-A 3300044 (maximale Propenlast = 112 Nl Propen/l·h), EP-A 575897 (maximale Propenlast = 120 Nl Propen/l·h), DE-C 3338380 (in im wesentlichen allen Beispielen beträgt die maximale Propenlast 126 Nl Propen/l·h; nur im Fall einer speziellen Katalysatorzusammensetzung wurde eine Propenlast von 162 Nl/l·h realisiert) und DE-A 19855913 (maximale Propenlast = 155 Nl Propen/l·h).

Die WO 98/24746 erachtet es bereits bei einer Propenbelastung von bis zu 148,8 Nl Propen/l·h als erforderlich, die Katalysatorschüttung so zu strukturieren, daß.ihre volumenspezifische Aktivität in Strömungsrichtung des Reaktionsgasgemisches sukzessive zunimmt.

Die JP-A 91/294239 offenbart zwar in einer beispielhaften Ausführungsform bei im wesentlichen konventioneller Verfahrensweise eine Propenlast der Katalysatorschüttung von 160 Nl Propen/l·h für eine katalytische Gasphasenoxidation von Propen zu Acrolein als möglich, dies jedoch ebenfalls nur zum Preis einer in Strömngsrichtung des Reaktionsgasgemisches sukzessive zunehmenden volumenspezifischen Aktivität. Eine solche Verfahrensweise ist großtechnisch aber nur wenig praktikabel, wird die gasphasenkatalytische Oxidation von Propen zu Acrolein üblicherweise doch in Rohrbündelreaktoren mit einigen tausend Kontaktrohren durchgeführt. von denen jedes einzelne mit der abgestuften Katalysatorschüttung beschickt werden muß.

Die EP-B 253409 und das zugehörige Äquivalent, die EP-B 257565, offenbaren, daß bei Verwendung eines inerten Verdünnungsgases das eine höhere molare Wärmekapazität als molekularer Stickstoff aufweist, der Anteil an Propen im Reaktionsgasausgangsgemisch erhöht werden kann. Nichtsdestotrotz liegt aber auch in den beiden vorgenannten Schriften die maximale realisierte Propenbelastung der

Katalysatorschüttung bei 140 Nl Propen/l·h.

Lediglich in der EP-A 293224 wurden bisher Propenbelastungen oberhalb von 160 Nl Propen/l·h realisiert. Dies allerdings auf Kosten eines speziellen zu verwendenden inerten Verdünnungsgases, das völlig frei von molekularem Stickstoff ist. Nachteilig an diesem Verdünnungsgas ist insbesondere, daß es sich bei all seinen Bestandteilen, im Unterschied zu molekularem Stickstoff, um Wertprodukte handelt, die bei einer kontinuierlichen Durchführung des Verfahrens in aufwendiger Weise aus Gründen der Wirtschaftlichkeit wenigstens teilweise in die Gasphasenoxidation rückgeführt werden müssen.

In der EP-B 450596 wurde unter Anwendung einer strukturierten Katalysatorschüttung eine Propenbelastung von 202,5 Nl Propen/l·h realisiert. Dies allerdings auf Kosten einer verringerten Wertproduktselektivität.

Die Aufgabe der vorliegenden Erfindung bestand daher darin, ein wie eingangs definiertes Verfahren der katalytischen Gasphasenoxidation von Propen zu Acrolein zur Verfügung zu stellen, das eine erhöhte Raum-Zeit-Ausbeute an Wertprodukt gewährleistet, ohne die Nachteile der Hochlastfahrweisen des Standes der Technik aufzuweisen.

Demgemäß wurde ein Verfahren der katalytischen Gasphasenoxidation von Propen zu Acrolein, bei dem man ein Propen, molekularen Sauerstoff und wenigstens ein Inertgas, das zu wenigstens 20 % seines Volumens aus molekularem Stickstoff besteht, enthaltendes Reaktionsgasausgangsgemisch, das den molekularen Sauerstoff und das Propen in einem molaren Verhältnis $O_2:C_3H_6 \geq 1$ enthält, bei erhöhter Temperatur so über einen Festbettkatalysator, dessen Aktivmasse wenigstens ein Molybdän und/oder Wolfram sowie Wismut, Tellur, Antimon, Zinn und/oder Kupfer (vorzugsweise wenigstens ein Mo, Bi und Fe) enthaltendes Multimetalloxid ist, führt, daß der Propenumsatz bei einmaligem Durchgang $\geq 90$ mol-% und die damit einhergehende Selektivität der Acroleinbildung sowie der Acrylsäurenebenproduktbildung zusammengenommen $\geq 90$ mol-% betragen, gefunden, das dadurch gekennzeichnet ist, daß

a) die Belastung des Festbettkatalysators mit dem im Reaktionsgasausgangsgemisch enthaltenen Propen $\geq 160$ Nl Propen/l Katalysatorschüttung · h beträgt,

b) der Festbettkatalysator aus einer in zwei räumlich aufeinanderfolgenden Reaktionszonen A,B angeordneten Katalysatorschüttung besteht, wobei die Temperatur der Reaktionszone A 300 bis 390°C (häufig bis 350°C) und die Temperatur der Reaktionszone B 305 bis 420°C (häufig bis zu 380°C), beträgt und gleichzeitig wenigstens 5°C oberhalb der Temperatur der Reaktionszone A liegt,

c) das Reaktionsgasausgangsgemisch die Reaktionszonen A,B in der zeitlichen Abfolge "erst A", "dann B" durchströmt und

d) sich die Reaktionszone A bis zu einem Umsatz des Propens von 40 bis 80 mol-% erstreckt.

Bevorzugt erstreckt sich die Reaktionszone A bis zu einem Propenumsatz von 50 bis 70 mol-% und besonders bevorzugt bis zu einem Propenumsatz von 65 bis 75 mol-%.

Die Temperatur der Reaktionszone B beträgt erfindungsgemäß in vorteilhafter Weise 305 bis 365°C, bzw. 340°C und besonders vorteilhaft 310 bis 330°C.

Ferner liegt die Temperatur der Reaktionszone B bevorzugt wenigstens 10°C oberhalb der Temperatur der Reaktionszone A.

Je höher die Propenbelastung der Katalysatorschüttung beim erfindungsgemäßen Verfahren gewählt wird, um so größer sollte die Differenz zwischen der Temperatur der Reaktionszone A und der Temperatur der Reaktionszone B gewählt werden. Normalerweise wird die vorgenannte Temperaturdifferenz beim erfindungsgemäßen Verfahren aber nicht mehr als 50°C betragen. D.h., die Differenz zwischen der Temperatur der Reaktionszone A und der Temperatur der Reaktionszone B kann erfindungsgemäß bis zu 20°C, bis zu 25°C, bis zu 30°C, bis zu 40°C, bis zu 45°C oder bis zu 50°C betragen.

In der Regel wird der auf den einfachen Durchgang bezogene Propenumsatz beim erfingungsgemäßen Verfahren $\geq$ 92 mol-% oder $\geq$ 94 mol-% betragen. Die Selektivität der Wertproduktbildung wird dabei regelmäßig $\geq$ 92 mol-% oder $\geq$ 94 mol-%, häufig $\geq$ 95 mol-% oder $\geq$ 96 mol-% bzw. $\geq$ 97 mol-% betragen.

In überraschender weise gilt das Vorgenannte nicht nur bei Propenbelastungen der Katalysatorschüttung von $\geq$ 165 Nl/l·h oder von $\geq$ 170 Nl/l·h bzw. $\geq$ 175 Nl/l·h oder $\geq$ 180 Nl/l·h, sondern auch bei Propenbelastungen der Katalysatorschüttung von $\geq$ 185 Nl/l·h oder $\geq$ 190 Nl/l·h bzw. $\geq$ 200 Nl/l·h oder $\geq$ 210 Nl/l·h sowie bei Belastungswerten $\geq$ 220 Nl/l·h oder $\geq$ 230 Nl/l·h bzw. $\geq$ 240 Nl/l·h oder $\geq$ 250 Nl/l·h.

Dabei überrascht, daß vorgenannte Werte selbst dann erreichbar sind, wenn das erfindungsgemäß verwendete Inertgas zu $\geq$ 30 Vol.-%, oder zu $\geq$ 40 Vol.-%, oder zu $\geq$ 50 Vol.-%, oder zu $\geq$ 60 Vol.-%, oder zu $\geq$ 70 Vol.-%, oder zu $\geq$ 80 Vol.-%, oder zu $\geq$ 90 Vol.-%, oder zu $\geq$ 95 Vol.-% aus molekularem Stickstoff besteht. Bei Propenbelastungen oberhalb von 250 Nl/l·h wird für das erfindungsgemäße Verfahren die Mitverwendung von inerten (inerte Verdünnungsgase sollen generell solche sein, die sich beim einmaligen Durchgang zu weniger als 5 %, bevorzugt zu weniger als 2 % umsetzen) Verdünnungsgasen wie Propan, Ethan, Methan, Pentan, Butan, $CO_2$, CO, Wasserdampf und/oder Edelgasen empfohlen. Selbstverständlich können diese Gase und ihre Gemische aber auch bereits bei geringeren Belastungen mitverwendet oder als alleinige Verdünnungsgase verwendet werden. Ferner überrascht, daß das erfindungsgemäße Verfahren mit einer über beide Reaktionszonen betrachtet homogenen, d.h., chemisch einheitlichen, Katalysatorschüttung durchgeführt werden kann, ohne in nennenswertem Umfang Umsatz- und/oder Selektivitätseinbußen zu erleiden.

Normalerweise wird beim erfindungsgemäßen Verfahren die Propenbelastung den Wert von 600 Nl/l·h nicht überschreiten. In typischer Weise liegen die Propenbelastungen beim erfindungsgemäßen Verfahren ohne nennenswerten Verlust von Umsatz und Selektivität bei Werten $\leq$ 300 Nl/l·h, häufig $\leq$ 250 Nl/l·h.

Der Arbeitsdruck kann beim erfindungsgemäßen Verfahren sowohl unterhalb von Normaldruck (z.B. bis zu 0,5 bar) als auch oberhalb von Normaldruck liegen. Typischerweise wird der Arbeitsdruck bei Werten von 1 bis 5 bar, häufig 1,5 bis 3,5 bar liegen. Normalerweise wird der Reaktionsdruck 100 bar nicht überschreiten.

Das molare Verhältnis von $O_2:C_3H_6$ im Reaktionsgasausgangsgemisch muß erfindungsgemäß $\geq$ 1 betragen. Üblicherweise wird dieses Verhältnis bei Werten $\leq$ 3 liegen. Häufig beträgt das molare Verhältnis von $O_2:C_3H_6$ im Reaktionsgasausgangsgemisch erfindungsgemäß $\geq$ 1,5 und $\leq$ 2,0.

Als Quelle für den im Rahmen des erfindungsgemäßen Verfahrens erforderlichen molekularen Sauerstoff kommt sowohl Luft, als auch an molekularem Stickstoff entreicherte Luft (z.B. $\geq$ 90 Vol.-% $O_2$, $\leq$ 10 Vol.-% $N_2$) in Betracht.

Der Propenanteil im Reaktionsgasausgangsgemisch kann erfindungsgemäß z.B. bei Werten von 4 bis 15 Vol.-%, häufig bei 5 bis 12 Vol.-% bzw. 5 bis 8 Vol.-% liegen (jeweils bezogen auf das Gesamtvolumen).

Häufig wird man das erfindungsgemäße Verfahren bei einem Propen:Sauerstoff:indifferente Gase (einschließlich Wasserdampf) Volumenverhältnis im Reaktionsgasausgangsgemisch von 1:(1,0 bis 3,0):(5 bis 25), vorzugsweise 1:(1,7 bis 2,3):(10 bis 15) durchführen.

Normalerweise enthält das Reaktionsgasausgangsgemisch neben den genannten Bestandteilen im wesentlichen keine weiteren Komponenten.

Als Festbettkatalysatoren kommen für das erfindungsgemäße Verfahren alle diejenigen in Betracht, deren Aktivmasse wenigstens ein Mo, Bi und Fe enthaltendes Multimetalloxid ist.

D.h., prinzipiell können alle diejenigen Katalysatoren, die in den Schriften DE-C 3338380, DE-A 19902562, EP-A 15565, DE-C 2830765, EP-A 807465, EP-A 279374, DE-A 3300044, EP-A 575897, US-A 4438217, DE-A 19855913, WO 98/24746, DE-A 19746210 (diejenigen der allgemeinen Formel II), JP-A 91/294239, EP-A 293224 und EP-A 700714 offenbart werden, erfindungsgemäß eingesetzt werden. Dies gilt insbesondere für die beispielhaf ten Ausführungsformen in diesen Schriften, unter denen jene der EP-A 15565, der EP-A 575897, der DE-A 19746210 und der DE-A 19855913 besonders bevorzugt werden. Besonders hervorzuheben sind in diesem Zusammenhang ein Katalysator gemäß Beispiel 1c aus der EP-A 15565 sowie ein in entsprechender weise herzustellender Katalysator, dessen Aktivmasse jedoch die Zusammensetzung $Mo_{12}Ni_{6,5}Zn_2Fe_2Bi_1P_{0,0065}K_{0,06}O_x \cdot 10SiO_2$ aufweist. Ferner sind hervorzuheben das Beispiel mit der laufenden Nr. 3 aus der DE-A 19855913 (Stöchiometrie: $Mo_{12}Co_7 Fe_3 Bi_{0,6}K_{0,08}Si_{1,6}O_x$) als Hohlzylindervollkatalysator der Geometrie 5 mm x 3 mm x 2 mm (Außendurchmesser x Höhe x Innendurchmesser) sowie der Multimetalloxid II - Vollkatalysator gemäß Beispiel 1 der DE-A 19746210. Ferner wären die Multimetalloxid-Katalysatoren der US-A 4438217 zu nennen. Letzteres gilt insbesondere dann, wenn diese Hohlzylinder eine Geometrie 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen.

Eine vielzahl der erfindungsgemäß geeigneten Multimetalloxidaktivmassen läßt sich unter der allgemeinen Formel I

$$Mo_{12}Bi_aFe_bX^1_cX^2_dX^3_eX^4_fO_n \qquad \text{(I)},$$

in der die Variablen nachfolgende Bedeutung aufweisen:

$X^1 =$      Nickel und/oder Kobalt,
$X^2 =$      Thallium, ein Alkalimetall und/oder ein Erdalkalimetall,
$X^3 =$      Zink, Phosphor, Arsen, Bor, Antimon, Zinn, Cer, Blei und/ oder Wolfram,
$X^4 =$      Silicium, Aluminium, Titan und/oder Zirkonium,
$a =$      0,5 bis 5,
$b =$      0,01 bis 5, vorzugsweise 2 bis 4,
$c =$      0 bis 10, vorzugsweise 3 bis 10,
$d =$      0 bis 2, vorzugsweise 0,02 bis 2,
$e =$      0 bis 8, vorzugsweise 0 bis 5,
$f =$      0 bis 10 und
$n =$      eine Zahl, die durch die Wertigkeit und Häufigkeit der von Sauerstoff verschiedenen Elemente in I bestimmt wird, subsummieren.

Sie sind in an sich bekannter Weise erhältlich (siehe z.B. die DE-A 4023239) und werden üblicherweise in Substanz zu Kugeln, Ringen oder Zylindern geformt oder auch in Gestalt von Schalenkatalysatoren, d.h., mit der Aktivmasse beschichteten vorgeformten, inerten Trägerkörpern, eingesetzt. Selbstverständlich können sie aber auch in Pulverform als Katalysatoren angewendet werden. Selbstverständlich kann erfindungsgemäß auch der Bi, Mo und Fe umfassende Multimetalloxidkatalysator ACS-4 der Fa. Nippon Schokubai verwendet werden.

Prinzipiell können als erfindungsgemäß geeignete Aktivmassen, insbesondere solche der allgemeinen Formel I, in einfacher Weise dadurch hergestellt werden, daß man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, $NH_3$, CO und/oder $H_2$) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen I kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, Ammonium-Salze und/oder Hydroxide in Betracht (Verbindungen wie $NH_4OH$, $(NH_4)_2CO_3$, $NH_4NO_3$, $NH_4CHO_2$, $CH_3COOH$, $NH_4CH_3CO_2$ und/oder Ammoniumoxalat, die spätestens beim späteren Calcinieren zu vollständig gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidmassen I kann in trockener oder in nasser Form erfolgen. Erfolgt er in trockener Form, so werden die Ausgangsverbindungen zweckmäßigerweise als feinteilige Pulver eingesetzt und nach dem Mischen und gegebenenfalls Verdichten der Calcinierung unterworfen. vorzugsweise erfolgt das innige Vermischen jedoch in nasser Form. Üblicherweise werden dabei die Ausgangsverbindungen in Form einer wäßrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wäßrige Masse getrocknet, wobei der Trocknungsprozeß vorzugsweise durch Sprühtrocknung der wäßrigen Mischung mit Austrittstemperaturen von 100 bis 150°C erfolgt.

Die erfindungsgemäß geeigneten Multimetalloxidmassen, insbesondere jene der allgemeinen Formel I, können für das erfindungsgemäße Verfahren sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Selbstverständlich kann die Formgebung der pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist. Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 μm, bevorzugt im Bereich 50 bis 500 μm und besonders bevorzugt im Bereich 150 bis 250 μm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit, deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 3 bis 6 mm. einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepaßt (vgl. EP-A 714 700).

Günstige erfindungsgemäß zu verwendende Multimetalloxidaktivmassen sind ferner Massen der allgemeinen Formel II

$$[Y^1_{a'}Y^2_{b'}O_{x'}]_p[Y^3_{c'}Y^4_{d'}Y^5_{e'}Y^6_{f'}Y^7_{g'}Y^2_{h'}O_{y'}]_q \qquad (II),$$

in der die Variablen folgende Bedeutung haben:

$Y^1$ =    Wismut, Tellur, Antimon, Zinn und/oder Kupfer,
$Y^2$ =    Molybdän und/oder Wolfram,
$Y^3$ =    ein Alkalimetall, Thallium und/oder Samarium,
$Y^4$ =    ein Erdalkalimetall, Nickel, Kobalt, Kupfer, Mangan, Zink, Zinn, Cadmium und/oder Quecksilber,
$Y^5$ =    Eisen, Chrom, Cer und/oder Vanadium,
$Y^6$ =    Phosphor, Arsen, Bor und/oder Antimon,
$Y^7$ =    ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

a' =    0,01 bis 8,
b' =    0,1 bis 30,
c' =    0 bis 4,
d' =    0 bis 20,
e' =    0 bis 20,
f' =    0 bis 6,
g' =    0 bis 15,
h' =    8 bis 16,
x',y' =    Zahlen, die durch die Wertigkeit und Häufigkeit der von Sauerstoff verschiedenen Elemente in II bestimmt werden und
p,q =    Zahlen, deren Verhältnis p/q 0,1 bis 10 beträgt,

enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Y^1_{a'}Y^2_{b'}O_{x'}$, deren Größtdurchmesser (längste durch den Schwerpunkt des Bereichs gehende Verbindungsstrecke zweier auf der Oberfläche (Grenzfläche) des Bereichs befindlicher Punkte) 1 nm bis 100 μm, häufig 10 nm bis 500 nm oder 1 μm bis 50 bzw. 25 μm, beträgt.

Besonders vorteilhafte erfindungsgemäße Multimetalloxidmassen II sind solche, in denen $Y^1$ Wismut ist.

Unter diesen werden wiederum jene bevorzugt, die der allgemeinen Formel III

$$[Bi_{a''}Z^2_{b''}O_{x''}]_{p''} \, [Z^2_{12}Z^3_{c''}Z^4_{d''}Fe_{e''}Z^5_{f''}Z^6_{g''}Z^7_{h''}O_{y''}]_{q''} \tag{III},$$

in der die Varianten folgende Bedeutung haben:

$Z^2$ = Molybdän und/oder Wolfram,
$Z^3$ = Nickel und/oder Kobalt,
$Z^4$ = Thallium, ein Alkalimetall und/oder ein Erdalkalimetall,
$Z^5$ = Phosphor, Arsen, Bor, Antimon, Zinn, Cer und/oder Blei,
$Z^6$ = Silicium, Aluminium, Titan und/oder Zirkonium,
$Z^7$ = Kupfer, Silber und/oder Gold,

$a''$ = 0,1 bis 1,
$b''$ = 0,2 bis 2,
$c''$ = 3 bis 10,
$d''$ = 0,02 bis 2,
$e''$ = 0,01 bis 5, vorzugsweise 0,1 bis 3,
$f''$ = 0 bis 5,
$g''$ = 0 bis 10,
$h''$ = 0 bis 1,
$x'',y''$ = Zahlen, die durch die Wertigkeit und Häufigkeit der von Sauerstoff verschiedenen Elemente in III bestimmt werden,
$p'',q''$ = Zahlen, deren Verhältnis $p''/q''$ 0,1 bis 5, vorzugsweise 0,5 bis 2 beträgt,

entsprechen, wobei diejenigen Massen III ganz besonders bevorzugt werden, in denen $Z^2_{b''}$ = (Wolfram)$_{b''}$ und $Z^2_{12}$ = (Molybdän)$_{12}$ ist.

Ferner ist es von Vorteil, wenn wenigstens 25 mol-% (bevorzugt wenigstens 50 mol-% und besonders bevorzugt wenigstens 100 mol-%) des gesamten Anteils $[y^1_{a'}y^2_{b'}O_{x'}]_p$ ($[Bi_{a''}Z^2_{b''}O_{x''}]_{p''}$) der erfindungsgemäß geeigneten Multimetalloxidmassen II (Multimetalloxidmassen III) in den erfindungsgemäß geeigneten Multimetalloxidmassen II (Multimetalloxidmassen III) in Form dreidimensional ausgehenter, von ihrer lokalen Umgebung aufgrund ihrer von ihrer lokalen Umgebung verschiedenen chemischen Zusammensetzung abgegrenzter, Bereiche der chemischen Zusammensetzung $y^1_{a'}y^2_{b'}O_{x'}$ $[Bi_{a}Z^2_{b''}O_{x''}]$ vorliegen, deren Größtdurchmesser im Bereich 1 nm bis 100 μm liegt.

Hinsichtlich der Formgebung gilt bezüglich Multimetalloxidmassen II-Katalysatoren das bei den Multimetalloxidmassen I-Katalysatoren Gesagte.

Die Herstellung von Multimetalloxidmassen II-Aktivmassen ist z.B. in der EP-A 575897 sowie in der DE-A 19855913 beschrieben.

Im anwendungstechnisch zweckmäßiger Weise erfolgt die Durchführung des erfindungsgemäßen Verfahrens in einem Zweizonenrohrbündelreaktor. Eine bevorzugte Variante eines erfindungsgemäß einsetzbaren Zweizonenrohrbündelreaktors offenbart die DE-C 2830765. Aber auch die in der DE-C 2513405, der US-A 3147084, der DE-A 2201528 und der DE-A 2903218 offenbarten Zweizonenrohrbündelreaktoren sind für eine Durchführung des erfindungsgemäßen Verfahrens geeignet.

D.h., in einfachster Weise befindet sich der erfindungsgemäß zu verwendende Festbettkatalysator in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Reaktionszone. D.h., in einfachster Weise umströmt ein Salzbad A denjenigen Abschnitt der Rohre (die Reaktionszone A), in welchem sich die oxidative Umsetzung des Propens (beim einfachen Durchgang) bis zum Erreichen eines Umsatzes im Bereich von 40 bis 80 mol-% vollzieht und ein Salzbad B umströmt den Abschnitt der Rohre (die Reaktionszone B), in welchem sich die oxidative Anschlußumsetzung des Propens (beim einfachen Durchgang) bis zum Erreichen eines Umsatzwertes von wenigstens 90 mol-% vollzieht (bei Bedarf können sich an die erfindungsgemäß anzuwendenden Reaktionszonen A,B weitere Reaktionszonen anschließen, die auf individuellen Temperaturen gehalten werden).

Anwendungstechnisch zweckmäßig umfaßt das erfindungsgemäße Verfahren keine weiteren Reaktionszonen. D.h., das Salzbad B umströmt zweckmäßig den Abschnitt der Rohre, in welchem sich die oxidative Anschlußumsetzung des Propens (beim einfachen Durchgang) bis zu einem Umsatzwert $\geq$ 92 mol-% oder $\geq$ 94 mol-% oder mehr vollzieht.

Üblicherweise liegt der Beginn der Reaktionszone B hinter dem Heißpunktmaximum der Reaktionszone A. Das Heißpunktmaximum der Reaktionszone B liegt normalerweise unterhalb der Heißpunktmaximaltemperatur der Reaktionszone A.

Die beiden Salzbäder A,B können erfindungsgemäß relativ zur Strömungsrichtung des durch die Reaktionsrohre strömenden Reaktionsgasgemisches im Gleichstrom oder im Gegenstrom durch den die Reaktionsrohre umgebenden Raum geführt werden. Selbstverständlich kann erfindungsgemäß auch in der Reaktionszone A eine Gleichströmung und in der Reaktionszone B eine Gegenströmung (oder umgekehrt) angewandt werden.

Selbstverständlich kann man in allen vorgenannten Fallkonstellationen innerhalb der jeweiligen Reaktionszone der, relativ zu den Reaktionsrohren, erfolgenden Parallelströmung der Salzschmelze noch eine Querströmung überlagern, so daß die einzelne Reaktionszone einem wie in der EP-A 700714 oder in der EP-A 700893 beschriebenen Rohrbündelreaktor entspricht und insgesamt im Längsschnitt durch das Kontaktrohrbündel ein mäanderförmiger

Strömungsverlauf des Wärmeaustauschmittels resultiert.

Zweckmäßigerweise wird das Reaktionsgasausgangsgemisch der Katalysatorbeschickung auf die Reaktionstemperatur vorerwärmt zugeführt.

Üblicherweise sind in den vorgenannten Rohrbündelreaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 22 bis 26 mm. Anwendungstechnisch zweckmäßig beläuft sich die im Rohrbündelbehälter untergebrachte Anzahl an Kontaktrohren auf wenigstens 5000, vorzugsweise auf wenigstens 10000. Käufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, daß der Abstand der zentrischen Innenachsen von zueinander nächstliegenden Kontaktrohren (die sogenannte Kontaktrohrteilung) 35 bis 45 mm beträgt (vgl. z.B. EP-B 468290).

Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweizonenrohrbündelreaktoren die Fließgeschwindigkeit innerhalb der beiden erforderlichen Wärmeaustauschmittelkreisläufen so gewählt, daß die Temperatur des Wärmeaustauschmittels von der Eintrittsstelle in die Reaktionszone bis zur Austrittstelle aus der Reaktionszone um 0 bis 15°C ansteigt. D.h., das vorgenannte ΔT kann erfindungsgemäß 1 bis 10°C, oder 2 bis 8°C oder 3 bis 6°C betragen.

Die Eintrittstemperatur des Wärmeaustauschmittels in die Reaktionszone A beträgt erfindungsgemäß normalerweise 300 bis 350°C. Die Eintrittstemperatur des Wärmeaustauschmittels in die Reaktionszone B beträgt erfindungsgemäß normalerweise einerseits 305 bis 380°C und liegt andererseits gleichzeitig wenigstens 5°C oberhalb der Eintrittstemperatur des in die Reaktionszone A eintretenden Wärmeaustauschmittels.

Bevorzugt liegt die Eintrittstemperatur des Wärmeaustauschmittels in die Reaktionszone B wenigstens 10°C oberhalb der Eintrittstemperatur des in die Reaktionszone A eintretenden Wärmeaustauschmittels. Die Differenz zwischen den Eintrittstemperaturen in die Reaktionszone A bzw. B kann erfindungsgemäß somit bis zu 20°C, bis zu 25°C, bis zu 30°C, bis zu 40°C, bis zu 45°C oder bis zu 50°C betragen. Normalerweise wird die vorgenannte Temperaturdifferenz aber nicht mehr als 50°C betragen. Je höher die Propenbelastung der Katalysatorschüttung beim erfindungsgemäßen Verfahren gewählt wird, um so größer sollte die Differenz zwischen der Eintrittstemperatur des Wärmeaustauschmittels in die Reaktionszone A und der Eintrittstemperatur des Wärmeaustauschmittels in die Reaktionszone B sein.

Mit Vorteil beträgt die Eintrittstemperatur des Wärmeaustauschmittels in die Reaktionszone B erfindungsgemäß 305 bis 365°C, bzw. 340°C und besonders vorteilhaft 310 bis 330°C.

Selbstverständlich können beim erfindungsgemäßen Verfahren die beiden Reaktionszonen A. B auch in räumlich voneinander getrennten Rohrbündelreaktoren realisiert sein. Bei Bedarf kann zwischen den beiden Reaktionszonen auch ein Wärmetauscher angebracht werden. Selbstredend können die beiden Reaktionszonen A, B auch als

Wirbelbett gestaltet werden.

Ferner können beim erfindungsgemäßen Verfahren auch Katalysatorschüttungen verwendet werden, deren volumenspezifische Aktivität in Strömungsrichtung des Reaktionsgemisches kontinuierlich, abrupt oder stufenförmig zunimmt (dies kann wie in der WO 98/24746 oder wie in der JP-A 91/294239 beschrieben oder auch durch Verdünnung mit Inertmaterial bewirkt werden). Ebenso können für die beschriebene Zweizonenfahrweise auch die in der EP-A 293224 und in der EP-B 257565 empfohlenen inerten Verdünnungsgase (z.H. nur Propan oder nur Methan etc.) eingesetzt werden. Letzteres bei Bedarf auch kombiniert mit einer in Strömungsrichtung des Reaktionsgasgemisches zunehmenden volumenspezifischen Aktivität der Katalysatorschüttung.

Es sei an dieser Stelle auch noch einmal darauf hingewiesen, daß für eine Durchführung des erfindungsgemäßen Verfahrens insbesondere auch der in der DE-AS 2201528 beschriebene Zweizonenrohrbündelreaktortyp verwendet werden kann, der die Möglichkeit beinhaltet, vom heißeren Wärmeaustauschmittel der Reaktionszone B eine Teilmenge an die Reaktionszone A abzuführen, um gegebenenfalls ein Anwärmen eines kalten Reaktionsgasausgangsgemisches oder eines kalten Kreisgases zu bewirken.

Das erfindungsgemäße Verfahren eignet sich insbesondere für eine kontinuierliche Durchführung. Es überrascht, daß es bei einmaligem Durchgang eine erhöhte Raum-Zeit-Ausbeute der Wertproduktbildung ermöglicht, ohne gleichzeitig die Selektivität der Wertproduktbildung nennenswert zu beeinträchtigen. Vielmehr wird in der Regel tendenziell sogar eine erhöhte Selektivität der wertproduktbildung beobachtet.

Letzteres ist vermutlich darauf zurückzuführen, daß das erfindungsgemäße verfahren aufgrund der im Bereich des erhöhten Propenumsatzes vorliegenden erhöhten Temperaturen eine geringere Readsorption des gebildeten Acroleins an den Festbettkatalysator bedingt.

Bemerkenswert ist ferner, daß die Katalysatorlebensdauer beim erfindungsgemäßen verfahren trotz der extremen Katalysatorbelastung mit Reaktanden im vollen Umfang zu befriedigen vermag.

Bei dem erfindungsgemäßen Verfahren wird kein reines Acrolein sondern ein Gemisch erhalten, von dessen Nebenkomponenten das Acrolein in an sich bekannter weise abgetrennt werden kann. Nicht umgesetztes Propen sowie verwendetes und/oder im Verlauf der Reaktion gebildetes inertes Verdünnungsgas können in die Gasphasenoxidation rückgeführt werden. Bei einer Verwendung des Acroleins zur Herstellung von Acrylsäure durch zweistufige katalytische Gasphasenoxidation von Propen werden die Acrolein enthaltenden Reaktionsgase in der Regel ohne Abtrennung der Nebenkomponenten in die zweite Oxidationsstufe überführt. Natürlich kann die erfindungsgemäße Zweizonenfahrweise bei Bedarf auch im Fall konventioneller Propenlasten angewendet werden.

Im übrigen sind in dieser Schrift Umsatz, Selektivität und Verweilzeit, falls nichts anderes erwähnt wird, wie folgt definiert:

$$\text{Umsatz } U_p \text{ an Propen (\%)} = \frac{\text{Molzahl umgesetztes Propen}}{\text{Molzahl eingeseiztes Propen}} \times 100$$

$$\text{Selektivität } S_A \text{ der Acroleinbildung (\%)} = \frac{\text{Molzahl Propen umgesetzt zu Acrolein}}{\text{Molzahl umgesetztes Propen}} \times 100$$

$$\text{Selektivität } S_{AS} \text{ der Acrylsäureneben-}$$

$$\text{produktbildung (\%)} = \frac{\text{Molzahl Propen umgesetzt zu Acrylsäure}}{\text{Molzahl umgesetztes Propen}} \times 100$$

$$\text{Selektivität } S_{WP} \text{ der Wertprodukt-}$$

$$\text{bildung (\%)} = \frac{\text{Molzahl Propen umgesetzt zu Acrolein und zu Acrylsäure}}{\text{Molzahl umgesetztes Propen}} \times 100$$

$$\text{Verweilzeit (sec)} = \frac{\text{mit Katalysator gefülltes Leervolumen des Reaktors (l)}}{\text{durchgesetztes Menge Reaktionsgasausgangsgemisch (l/h)}} \times 3600$$

Beispiele

a) Katalysatorherstellung

1. Herstellung einer Ausgangsmasse 1

In 775 kg einer wäßrigen salpetersauren Wismutnitratlösung (11,2 Gew.-% Bi, freie Salpetersäure 3 bis 5 Gew.-%; Massendichte: 1,22 bis 1,27 g/ml) wurden bei 25°C portionsweise 209,3 kg Wolframsäure (72,94 Gew.-% W) eingerührt. Das resultierende wäßrige Gemisch wurde anschließend noch 2 h bei 25°C gerührt und anschließend sprühgetrocknet.

Die Sprühtrocknung erfolgte in einem Drehscheibensprühturm im Gegenstrom bei einer Gaseintrittstemperatur von $300 \pm 10°C$ und einer Gasaustrittstemperatur von $100 \pm 10°C$. Das erhaltene Sprühpulver wurde anschließend bei einer Temperatur im Bereich von 780 bis 810°C calciniert (im luftdurchströmten Drehrohrofen (1,54 m$^3$ Innenvolumen, 200 Nm$^3$ Luft/h)). wesentlich bei der genauen Einstellung der Calcinationstemperatur ist, daß sie an der angestrebten Phasenzusammensetzung des Calcinationsprodukts orientiert zu erfolgen hat. Gewünscht sind die Phasen $WO_3$ (monoklin) und $Bi_2W_2O_9$, unerwünscht ist das Vorhandensein von $\gamma\text{-}Bi_2WO_6$ (Russellit). Sollte daher nach der

Calcination die Verbindung $\gamma$-$Bi_2WO_6$ anhand eines Reflexes im Pulverröntgendiffraktogramm bei einem Reflexwinkel von $2\ominus = 28,4°$ (CuK$\alpha$-Strahlung) noch nachweisbar sein, so ist die Präparation zu wiederholen und die Calcinationstemperatur innerhalb des angegebenen Temperaturbereichs zu erhöhen, bis das Verschwinden des Reflexes erreicht wird. Das so erhaltene vorgebildete calcinierte Mischoxid wurde gemahlen, so daß der $X_{50}$-Wert (vgl. Ullmann's Encyclopedia of Industrial Chemistry, 6th Edition (1998) Electronic Release, Kapitel 3.1.4 oder DIN 66141) der resultierenden Körnung 5 µm betrug. Das Mahlgut wurde dann mit 1 Gew.-% (bezogen auf das Mahlgut) feinteiligem $SiO_2$ (Rüttelgewicht 150 g/l; $X_{50}$-Wert der $SiO_2$-Partikel betrug 10 µm, die BET-Oberfläche betrug 100 $m^2$/g) gemischt.

2. Herstellung einer Ausgangsmasse 2

Eine Lösung A wurde hergestellt indem man bei 60°C unter Rühren in 600 l Wasser 213 kg Ammoniumheptamolybdat löste und die resultierende Lösung unter Aufrechterhaltung der 60°C und Rühren mit 0,97 kg einer 20°C aufweisenden wäßrigen Kaliumhydroxidlösung (46,8 Gew.-% KOH) versetzte.

Eine Lösung B wurde hergestellt indem man bei 60°C in 262,9 kg einer wäßrigen Cobaltnitratlösung (12,4 Gew.-% Co) 116,25 kg einer wäßrigen Eisennitratlösurg (14,2 Gew.-% Fe) eintrug. Anschließend wurde unter Aufrechterhaltung der 60°C die Lösung B über einen Zeitraum von 30 Minuten kontinuierlich in die vorgelegte Lösung A gepumpt. Anschließend wurde 15 Minuten bei 60°C gerührt. Dann wurden dem resultierenden wäßrigen Gemisch 19,16 kg eines Kieselgels (46,80 Gew.-% $SiO_2$, Dichte: 1,36 bis 1,42 g/ml, pH 8,5 bis 9,5, Alkaligehalt max. 0,5 Gew.-%) zugegeben und danach noch weitere 15 Minuten bei 60°C gerührt.

Anschließend wurde in einem Drehscheibensprühturm im Gegenstrom sprühgetrocknet (Gaseintrittstemperatur: 400± 10°C, Gasaustrittstemperatur: 140± 5°C). Das resultierende Sprühpulver wies einen Glühverlust von ca. 30 Gew.-% auf (3 h bei 600°C glühen).

3. Herstellung der Multimetalloxidaktivmasse

Die Ausgangsmasse 1 wurde mit der Ausgangsmasse 2 in der für eine Multimetalloxidaktivmasse der Stöchiometrie

$$[Bi_2W_2O_9 \cdot 2WO_3]_{0,5}[Mo_{12}Co_{5,5}Fe_{2,94}Si_{1,59}K_{0,08}O_x]_1$$

erforderlichen Menge homogen vermischt. Bezogen auf die vorgenannte Gesamtmasse wurden zusätzlich 1,5 Gew.-% feinteiliges Graphit (Siebanalyse: min. 50 Gew.-% < 24 µm, max. 10 Gew.-% > 24 µm und < 48 µm, max. 5 Gew.-% > 48 µm, BET-Oberfläche: 6 bis 13 $m^2$/g) homogen eingemischt. Das resultierende Trockengemisch wurde zu Hohlzylindern mit 3 mm Länge, 5 mm Außendurchmesser und 1,5 mm Wandstärke verpreßt und anschließend wie folgt thermisch behandelt.

Im Luft durchströmten Muffelofen (60 l Innenvolumen, 1 l/h Luft pro Gramm Aktivmassevorläufermasse) wurde mit einer Aufheizrate von 180°C/h zunächst von Raumtemperatur (25°C) auf 190°C aufgeheizt. Diese Temperatur wurde für 1 h aufrechterhalten und dann mit einer Aufheizrate von 60°C/h auf 210°C erhöht. Die 210°C wurden wiederum während 1 h aufrechterhalten, bevor sie mit einer Aufheizrate von 60°C/h, auf 230°C erhöht wurde. Diese Temperatur wurde ebenfalls 1 h aufrechterhalten bevor sie, wiederum mit einer Aufheizrate von 60°C/h, auf 265°C erhöht wurde. Die 265°C wurden anschließend ebenfalls während 1 h aufrechterhalten. Danach wurde zunächst auf Raumtemperatur abgekühlt und damit die Zersetzungsphase im wesentlichen abgeschlossen. Dann wurde mit einer Aufheizrate von 180°C/h auf 465°C erhitzt und diese Calcina-tionstemperatur während 4 h aufrechterhalten.

Die resultierenden Vollkatalysatorringe wurden für die nachfolgend beschriebene katalytische Gasphasenoxidation des Propens verwendet.

b) Gasphasenkatalytische Oxidation von Propen zu Acrolein

1. Beschickung des Reaktionsrohres

Ein Reaktionsrohr (V2A Stahl; 30 mm Außendurchmesser; 2 mm Wandstärke; 26 mm Innendurchmesser, Länge: 439 cm, sowie ein in der Reaktionsrohrmitte zentriertes Thermorohr (4 mm Außendurchmesser) zur Aufnahme eines Thermoelements mit dem die Temperatur im Reaktionsrohr ermittelt werden kann) wurde von unten nach oben auf einem Kontaktstuhl (44 cm Länge) zunächst auf einer Länge von 30 cm mit eine rauhe Oberfläche aufweisenden Steatitkugeln (4 bis 5 mm Durchmesser; Inertmaterial zum Erwärmen des Reaktionsgasausgangsgemisches) und anschließend auf einer Länge von 300 cm mit den in a) hergestellten Vollkatalysatorringen beschickt, bevor die Beschickung auf einer Länge von 30 cm mit den vorgenannten Steatitkugeln als Nachschüttung abgeschlossen wurde.

Die verbleibenden 35 cm Kontaktrohr wurden leer belassen.

Der Teil des Reaktionsrohres, der mit Feststoff beschickt war wurde mittels 12 zylinderförmig um das Rohr aufgegossenen Aluminium-Blöcken von je 30 cm Länge, die durch elektrische Heizbänder beheizt wurden, thermostatisiert (Vergleichsversuche mit einem entsprechenden mittels eines stickstoffgeperlten Salzbades beheizten Reaktionsrohr zeigten, daß die Aluminiumblock-Thermostatisierung eine Salzbad-Thermostatisierung zu simulieren vermag). Die ersten sechs Aluminumblöcke in Strömungsrichtung definierten eine Reaktionszone A und die verbleibenden sechs Aluminiumblöcke definierten eine Reaktionszone B. Die an Feststoff freien Enden des Reaktionsrohres wurden mit unter erhöhtem Druck befindlichem Wasserdampf auf 220°C gehalten.

Das vorstehend beschriebene Reaktionsrohr wurde mit einem Reaktionsgasausgangsgemisch der nachfolgenden Zusammensetzung kontinuierlich beschickt, wobei die Belastung und die Thermostatisierung des Reaktionsrohres variiert wurden:

6 bis 6,5 Vol-% Propen,
3 bis 3,5 Vol-% $H_2O$,
0,3 bis 0,5 Vol.-% CO,
0,8 bis 1,2 Vol-% $CO_2$,
0,025 bis 0,04 vol.-% Acrolein,
10,4 bis 10,7 Vol.-% $O_2$ und als Restmenge ad 100 % molekularer Stickstoff (Sauerstoffquelle war, abgesehen von geringem $O_2$-Gehalt im Kreisgas. Luft)

Dem Produktgasgemisch wurde am Reaktionsrohrausgang eine kleine Probe für eine gaschromatographische Analyse entnommen. Im übrigen wurde das Produktgasgemisch direkt in eine nachfolgende Acroleinoxidationsstufe (zu Acrylsäure) geführt. Vom Produktgasgemisch der Acroleinoxidationsstufe wurde die Acrylsäure in an sich bekannter Weise abgetrennt und ein Teil des verbleibenden Restgases zur Beschickung der Propenoxidationsstufe wiederverwendet (als sogenanntes "Kreisgas"), was den Acroleingehalt des vorgenannten Beschickungsgases und die geringe Varianz der Feedzusammensetzung erklärt.

Der Druck am Reaktionsrohreingang variierte in Abhängigkeit von der gewählten Propenbelastung im Bereich von 3,0 bis 1,9 bar. Am Ende der Reaktionszone A befand sich ebenfalls eine Analysenstelle.

Die in Abhängigkeit von der gewählten Propenbelastung und der gewählten Aluminium-Thermostatisierung erzielten Ergebnisse zeigt die nachfolgende Tabelle 1.

$T_A$ steht für die Temperatur der Aluminiumblöcke in der Reaktionszone A und $T_B$ steht für die Temperatur der Aluminiumblöcke in der Reaktionszone B.

$U_{PA}$ ist der Propenumsatz am Ende der Reaktionszone A und $U_{PE}$ ist der Propenumsatz am Reaktionsrohrausgang. $S_{AE}$, $S_{ASE}$ und $S_{WPE}$ sind die Selektivitäten $S_A$, $S_{AS}$ und $S_{WP}$ am Reaktionsrohrausgang und $RZA_{WP}$ ist die Raum-Zeit-Ausbeute an Wertprodukt am Reaktionsrohrausgang.

Tabelle 1

| Propenbelastung [Nl Propen/l·h] | $T_A$ [°C] | $T_B$ [°C] | $U_{PA}$ (%) | $U_{PE}$ (%) | $S_{AE}$ (%) | $S_{ASE}$ (%) | $S_{WPE}$ (%) | $RZA_{WP}$ (g/l·h) |
|---|---|---|---|---|---|---|---|---|
| 100 | 312 | 312 | 76,9 | 94,6 | 91,82 | 4,44 | 96,3 | 230,8 |
| 125 | 316 | 316 | 78,2 | 94,1 | 92,73 | 5,09 | 97,8 | 292,2 |
| 175 | 325 | 336 | 76,3 | 94,9 | 90,07 | 7,34 | 97,4 | 413,5 |
| 175 | 320 | 341 | 72,1 | 95,0 | 90,13 | 7,24 | 97,4 | 474,2 |
| 200 | 325 | 346 | 73,7 | 94,5 | 90,65 | 7,49 | 98,1 | 468,8 |

Erhöht man die Propenlast auf Werte > 200 Nl Propen/l·h, so erhält man die Ergebnisse gemäß Tabelle 2. Zusätzlich enthält Tabelle 2 die einzustellenden Bedingungen für einen Vergleichsversuch bei 200°C.

Tabelle 2

| Propenbelastung [Nl Propen/l·h] | $T_A$ [°C] | $T_B$ [°C] | $U_{PA}$ (%) | $U_{PE}$ (%) | $S_{AE}$ (%) | $S_{ASE}$ (%) | $S_{WPE}$ (%) | $RZA_{WP}$ (g/l·h) |
|---|---|---|---|---|---|---|---|---|
| 225 | 325 | 357 | 72,4 | 94,5 | 89,22 | 7,88 | 97,1 | 528,6 |
| 250 | 330 | 361 | 72,3 | 94,5 | 88,84 | 8,07 | 96,91 | 586,5 |
| 200 (Vergleichsversuch) | 300 | 370 | 44 | 94,5 | 88,2 | 8,5 | 96,7 | 468,8 |

EP 1 159 244 B1

**Patentansprüche**

1. Verfahren der katalytischen Gasphasenoxidation von Propen zu Acrolein, bei dem man ein Propen, molekularen Sauerstoff und wenigstens ein Inertgas, das zu wenigstens 20 % seines Volumens aus molekularem Stickstoff besteht, enthaltendes Reaktionsgasausgangsgemisch, das den molekularen Sauerstoff und das Propen in einem molaren Verhältnis $O_2:C_3H_6 \geq 1$ enthält, bei erhöhter Temperatur so über einen Festbettkatalysator, dessen Aktivmasse wenigstens ein Molybdän und/oder Wolfram sowie Wismut, Tellur, Antimon, Zinn und/oder Kupfer enthaltendes Multimetalloxid ist, führt, daß der Propenumsatz bei einmaligem Durchgang ≥ 90 mol-% und die damit einhergehende Selektivität der Acroleinbildung sowie der Arylsäurenebenproduktbildung zusammengenommen ≥ 90 mol-% betragen, **dadurch gekennzeichnet, daß**

   a) die Belastung des Festbettkatalysators mit dem im Reaktionsgasausgangsgemisch enthaltenen Propen ≥ 160 Nl Propen/l Katalysatorschüttung · h beträgt,

   b) der Festbettkatalysator aus einer in zwei räumlich aufeinanderfolgenden Reaktionszonen A,B angeordneten Katalysatorschüttung besteht, wobei die Temperatur der Reaktionszone A 300 bis 390°C und die Temperatur der Reaktionszone B 305 bis 420°C beträgt und gleichzeitig wenigstens 5°C oberhalb der Temperatur der Reaktionszone A liegt,

   c) das Reaktionsgasausgangsgemisch die Reaktionszonen A,B in der zeitlichen Abfolge "erst A", "dann B" durchströmt und

   d) sich die Reaktionszone A bis zu einem Umsatz des Propens von 40 bis 80 mol-% erstreckt.

2. Verfahren nach Anspruch 1, **dadurch gekennzeichnet, daß** sich die Reaktionszone A bis zu einem Umsatz des Propens von 50 bis 70 mol-% erstreckt.

3. Verfahren nach Anspruch 1, **dadurch gekennzeichnet, daß** sich die Reaktionszone A bis zu einem Umsatz des Propens von 65 bis 75 mol-% erstreckt.

4. Verfahren nach einem der Ansprüche 1 bis 3, **dadurch gekennzeichnet, daß** die Temperatur der Reaktionszone B wenigstens 10°C oberhalb der Temperatur der Reaktionszone A liegt.

5. Verfahren nach einem der Ansprüche 1 bis 4, **dadurch gekennzeichnet, daß** die Temperatur der Reaktionszone B 305 bis 340°C beträgt.

6. Verfahren nach einem der Ansprüche 1 bis 4, **dadurch gekennzeichnet, daß** die Temperatur der Reaktionszone B 310 bis 330°C beträgt.

7. Verfahren nach einem der Ansprüche 1 bis 6, **dadurch gekennzeichnet, daß** der Propenumsatz bei einmaligem Durchgang ≥ 94 mol-% beträgt.

8. Verfahren nach einem der Ansprüche 1 bis 7, **dadurch gekennzeichnet, daß** die Selektivität der Acroleinbildung sowie der Acrylsäurenebenproduktbildung zusammengenommen ≥ 94 mol-% beträgt.

9. Verfahren nach einem der Ansprüche 1 bis 8, **dadurch gekennzeichnet, daß** die Propenbelastung der Katalysatorschüttung ≥ 165 Nl/l·h beträgt.

10. Verfahren nach einem der Ansprüche 1 bis 8, **dadurch gekennzeichnet, daß** die Propenbelastung der Katalysatorschüttung ≥ 170 Nl/l·h beträgt.

11. Verfahren nach einem der Ansprüche 1 bis 10, **dadurch gekennzeichnet, daß** das wenigstens eine Inertgas zu ≥ 40 Vol.-% aus molekularem Stickstoff besteht.

12. Verfahren nach einem der Ansprüche 1 bis 10, **dadurch gekennzeichnet, daß** das wenigstens eine Inertgas zu ≥ 60 Vol.-% aus molekularem Stickstoff besteht.

13. Verfahren nach einem der Ansprüche 1 bis 12, **dadurch gekennzeichnet, daß** das wenigstens eine Inertgas Wasserdampf umfaßt.

14. Verfahren nach einem der Ansprüche 1 bis 13, **dadurch gekennzeichnet, daß** das wenigstens eine Inertgas $CO_2$ und/oder CO umfaßt.

15. Verfahren nach einem der Ansprüche 1 bis 14, **dadurch gekennzeichnet, daß** es bei einem Arbeitsdruck von 0,5 bis 3,5 bar durchgeführt wird.

16. Verfahren nach einem der Ansprüche 1 bis 15, **dadurch gekennzeichnet, daß** das molare Verhältnis $O_2:C_3H_6$ im Reaktionsgasausgangsgemisch 1,5 bis 2,0 beträgt.

17. Verfahren nach einem der Ansprüche 1 bis 16, **dadurch gekennzeichnet, daß** als Sauerstoffquelle Luft mitverwendet wird.

18. Verfahren nach einem der Ansprüche 1 bis 17, **dadurch gekennzeichnet, daß** der Propengehalt des Reaktionsgasausgangsgemisches 4 bis 15 Vol.-% beträgt.

19. Verfahren nach einem der Ansprüche 1 bis 17, **dadurch gekennzeichnet, daß** der Propengehalt des Reaktionsgasausgangsgemisches 5 bis 12 Vol.-% beträgt.

20. Verfahren nach einem der Ansprüche 1 bis 17, **dadurch gekennzeichnet, daß** der Propengehalt des Reaktionsgasausgangsgemisches 5 bis 8 Vol.-% beträgt.

21. Verfahren nach einem der Ansprüche 1 bis 20, **dadurch gekennzeichnet, daß** die Aktivmasse des Festbettkatalysators wenigstens ein Multimetalloxid der allgemeinen Formel I

$$Mo_{12}Bi_aFe_bX_c^1X_d^2X_e^3X_f^4O_n \qquad (I)$$

ist, in der die Variablen nachfolgende Bedeutung aufweisen:

$X^1 =$ Nickel und/oder Kobalt,
$X^2 =$ Thallium, ein Alkalimetall und/oder ein Erdalkalimetall,
$X^3 =$ Zink, Phosphor, Arsen, Bor, Antimon, Zinn, Cer, Blei und/oder Wolfram,
$X^4 =$ Silicium, Aluminium, Titan und/oder Zirkonium,
a = 0,5 bis 5,
b = 0,01 bis 5,
c = 0 bis 10,
d = 0 bis 2,
e = 0 bis 8,
f = 0 bis 10 und
n = eine Zahl, die durch die Wertigkeit und Häufigkeit der von Sauerstoff verschiedenen Elemente in I bestimmt wird.

22. Verfahren nach einem der Ansprüche 1 bis 20, **dadurch gekennzeichnet, daß** die Aktivmasse des Festbettkatalysators wenigstens ein Multimetalloxid der allgemeinen Formel II

$$[Y1_{a'}^1Y_{b'}^2O_{x'}]_p[Y_{c'}^3Y_{d'}^4Y_{e'}^5Y_{f'}^6Y_{g'}^7Y_{h'}^2O_{y'}^7]_q \qquad (II),$$

ist, in der die Variablen folgende Bedeutung haben:

$Y^1 =$ Wismut, Tellur, Antimon, Zinn und/oder Kupfer,
$Y^2 =$ Molybdän und/oder Wolfram,
$Y^3 =$ ein Alkalimetall, Thallium und/oder Samarium,
$Y^4 =$ ein Erdalkalimetall, Nickel, Kobalt, Kupfer, Mangan, Zink, Zinn, Cadmium und/oder Quecksilber,
$Y^5 =$ Eisen, Chrom, Cer und/oder Vanadium,

$Y^6$ = Phosphor, Arsen, Bor und/oder Antimon,

$Y^7$ = ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

a' = 0,01 bis 8,

b' = 0,1 bis 30,

c' = 0 bis 4,

d' = 0 bis 20,

e' = 0 bis 20,

f' = 0 bis 6,

g' = 0 bis 15,

h' = 8 bis 16,

x',y' = Zahlen, die durch die Wertigkeit und Häufigkeit der von Sauerstoff verschiedenen Elemente in II bestimmt werden und

p,q = Zahlen, deren Verhältnis p/q 0,1 bis 10 beträgt,

enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Y^1_{a'}Y^2_{b'}O_{x'}$, deren Größtdurchmesser 1 nm bis 100 µm betragen.

23. Verfahren nach einem der Ansprüche 1 bis 22, **dadurch gekennzeichnet, daß** die Katalysatorschüttung ring- und/oder kugelförmige Katalysatoren umfaßt.

24. Verfahren nach Anspruch 23, **dadurch gekennzeichnet, daß** die Ringgeometrie die folgende ist:

Außendurchmesser: 2 bis 10 mm,

Länge: 2 bis 10 mm,

Wandstärke: 1 bis 3 mm.

25. Verfahren nach Anspruch 23, **dadurch gekennzeichnet, daß** der kugelförmige Katalysator ein Schalenkatalysator bestehend aus einem kugelförmigen Träger (1 bis 8 mm Durchmesser) und einer auf diesem aufgebrachten Aktivmassenschale (10 bis 1000 µm Dicke) ist.

26. Verfahren nach einem der Ansprüche 1 bis 25, **dadurch gekennzeichnet, daß** es in einem Zweizonenrohbündelreaktor durchgeführt wird.

27. Verfahren nach einem der Ansprüche 1 bis 26, **dadurch gekennzeichnet, dass** die Temperatur der Reaktionszone A 300 bis 350°C und die Temperatur der Reaktionszone B 305 bis 380°C beträgt.

28. Verfahren nach einem der Ansprüche 1 bis 27, **dadurch gekennzeichnet, dass** der Propenumsatz bei einmaligem Durchgang ≥ 92 mol-% beträgt.

29. Verfahren nach einem der Ansprüche 1 bis 28, **dadurch gekennzeichnet, dass** der Festbettkatalysator eine Katalysatorschüttung ist, deren volumenspezifische Aktivität in Strömungsrichtung des Reaktionsgemisches durch Verdünnung mit Inertmaterial kontinuierlich, abrupt oder stufenförmig zunimmt.

30. Verfahren zur Herstellung von Acrylsäure, **dadurch gekennzeichnet, dass** es ein Verfahren gemäß einem der Ansprüche 1 bis 30 umfaßt.

## Claims

1. A process for the catalytic gas-phase oxidation of propene to acrolein, in which a reaction gas starting mixture comprising propene, molecular oxygen and at least one inert gas, at least 20% by volume of which consists of molecular nitrogen, and containing the molecular oxygen and the propene in a molar ratio $O_2:C_3H_6$ of ≥ 1 is passed, at elevated temperatures, over a fixed-bed catalyst, whose active material is at least one molybdenum- and/or tungsten- and bismuth-, tellurium-, antimony-, tin- and/or copper-containing multimetal oxide, in such a way that the propene conversion in a single pass is ≥ 90 mol% and the associated selectivity of the acrolein formation and of the acrylic acid byproduct formation together is $\geq$ 90 mol%, wherein

a) the loading of the fixed-bed catalyst with the propene contained in the reaction gas starting mixture is $\geq$ 160 l(S.T.P.) of propene per l of catalyst bed per h,

b) the fixed-bed catalyst consists of a catalyst bed arranged in two spatially successive reaction zones A, B, the temperature of the reaction zone A being from 300 to 390°C and the temperature of the reaction zone B being from 305 to 420°C and at the same time being at least 5°C above the temperature of the reaction zone A,

c) the reaction gas starting mixture flows first through the reaction zone A and then through the reaction zone B and

d) the reaction zone A extends to a propene conversion of from 40 to 80 mol%.

2.  A process as claimed in claim 1, wherein the reaction zone A extends to a propene conversion of from 50 to 70 mol%.

3.  A process as claimed in claim 1, wherein the reaction zone A extends to a propene conversion of from 65 to 75 mol%.

4.  A process as claimed in any of claims 1 to 3, wherein the temperature of the reaction zone B is at least 10°C above the temperature of the reaction zone A.

5.  A process as claimed in any of claims 1 to 4, wherein the temperature of the reaction zone B is from 305 to 340°C.

6.  A process as claimed in any of claims 1 to 4, wherein the temperature of the reaction zone B is from 310 to 330°C.

7.  A process as claimed in any of claims 1 to 6, wherein the propene conversion in a single pass is $\geq$ 94 mol%.

8.  A process as claimed in any of claims 1 to 7, wherein the selectivity of the acrolein formation and of the formation of acrylic acid byproduct together is $\geq$ 94 mol%.

9.  A process as claimed in any of claims 1 to 8, wherein the propene loading of the catalyst bed is $\geq$ 165 l(S.T.P.)/l·h.

10. A process as claimed in any of claims 1 to 8, wherein the propene loading of the catalyst bed is $\geq$ 170 l(S.T.P.)/l·h.

11. A process as claimed in any of claims 1 to 10, wherein the one or more inert gases comprise $\geq$ 40% by volume of molecular nitrogen.

12. A process as claimed in any of claims 1 to 10, wherein the one or more inert gases comprise $\geq$ 60% by volume of molecular nitrogen.

13. A process as claimed in any of claims 1 to 12, wherein the one or more inert gases comprise steam.

14. A process as claimed in any of claims 1 to 13, wherein the one or more inert gases comprise $CO_2$ and/or CO.

15. A process as claimed in any of claims 1 to 14, which is carried out at an operating pressure of from 0.5 to 3.5 bar.

16. A process as claimed in any of claims 1 to 15, wherein the molar $O_2$:$C_3H_6$ ratio in the reaction gas starting mixture is from 1.5 to 2.0.

17. A process as claimed in any of claims 1 to 16, wherein air is concomitantly used as the oxygen source.

18. A process as claimed in any of claims 1 to 17, wherein the propene content of the reaction gas starting mixture is from 4 to 15% by volume.

19. A process as claimed in any of claims 1 to 17, wherein the propene content of the reaction gas starting mixture is from 5 to 12% by volume.

**20.** A process as claimed in any of claims 1 to 17, wherein the propene content of the reaction gas starting mixture is from 5 to 8% by volume.

**21.** A process as claimed in any of claims 1 to 20, wherein the active material of the fixed-bed catalyst is at least one multimetal oxide of the formula I

$$Mo_{12}Bi_aFe_bX^1_cX^2_dX^3_eX^4_fO_n \tag{I}$$

where

| | |
|---|---|
| $X^1$ | is nickel and/or cobalt, |
| $X^2$ | is thallium, an alkali metal and/or an alkaline earth metal, |
| $X^3$ | is zinc, phosphorus, arsenic, boron, antimony, tin, cerium, lead and/or tungsten, |
| $X^4$ | is silicon, aluminum, titanium and/or zirconium, |
| a | is from 0.5 to 5, |
| b | is from 0.01 to 5, |
| c | is from 0 to 10, |
| d | is from 0 to 2, |
| e | is from 0 to 8, |
| f | is from 0 to 10 and |
| n | is a number which is determined by the valency and frequency of the elements in I other than oxygen. |

**22.** A process as claimed in any of claims 1 to 20, wherein the active material of the fixed-bed catalyst is at least one multimetal oxide of the formula II

$$[Y^1_{a'}Y^2_{b'}O_{x'}]_p(Y^3_{c'}Y^4_{d'}Y^5_{e'}Y^6_{f'}Y^7_{g'}Y^2_{h'}O_{y'}]_q \tag{II},$$

where

| | |
|---|---|
| $Y^1$ | is bismuth, tellurium, antimony, tin and/or copper, |
| $Y^2$ | is molybdenum and/or tungsten, |
| $Y^3$ | is an alkali metal, thallium and/or samarium, |
| $Y^4$ | is an alkaline earth metal, nickel, cobalt, copper, manganese, zinc, tin, cadmium and/or mercury, |
| $Y^5$ | is iron, chromium, cerium and/or vanadium, |
| $Y^6$ | is phosphorus, arsenic, boron and/or antimony, |
| $Y^7$ | is a rare earth metal, titanium, zirconium, niobium, tantalum, rhenium, ruthenium, rhodium, silver, gold, aluminum, gallium, indium, silicon, germanium, lead, thorium and/or uranium, |
| a' | is from 0.01 to 8, |
| b' | is from 0.1 to 30, |
| c' | is from 0 to 4, |
| d' | is from 0 to 20, |
| e' | is from 0 to 20, |
| f' | is from 0 to 6, |
| g' | is from 0 to 15, |
| h' | is from 8 to 16, |
| x' and y' | are numbers determined by the valency and frequency of the elements in II other than oxygen and |
| p and q | are numbers whose ratio p/q is from 0.1 to 10, |

containing three-dimensional regions of the chemical composition $Y^1_aY^2_bO_x$, which are delimited from their local environment owing to their composition differing from their local environment and whose maximum diameters are from 1 nm to 100 μm.

**23.** A process as claimed in any of claims 1 to 22, wherein the catalyst bed comprises annular and/or spherical catalysts.

**24.** A process as claimed in claim 23, wherein the ring geometry is the following:

| external diameter | from 2 to 10 mm, |
|---|---|
| length | from 2 to 10 mm, |
| wall thickness | from 1 to 3 mm. |

**25.** A process as claimed in claim 23, wherein the spherical catalyst is a coated catalyst consisting of a spherical carrier (from 1 to 8 mm diameter) and a coat (from 10 to 1000 $\mu$m thick) comprising active material and applied to said carrier.

**26.** A process as claimed in any of claims 1 to 25, which is carried out in a two-zone tube-bundle reactor.

**27.** A process as claimed in any of claims 1 to 26, wherein the temperature of the reaction zone A is from 300 to 350°C and the temperature of the reaction zone B is from 305 to 380°C.

**28.** A process as claimed in any of claims 1 to 27, wherein the propene conversion in a single pass is $\geq$ 92 mol%.

**29.** A process as claimed in any of claims 1 to 28, wherein the fixed-bed catalyst is a catalyst bed whose volume-specific activity increases continuously, abruptly or stepwise in the direction of flow of the reaction mixture through dilution with inert material.

**30.** A process for the preparation of acrylic acid, which comprises a process as claimed in any of claims 1 to 29.

**Revendications**

**1.** Procédé d'oxydation catalytique en phase gazeuse de propène pour former de l'acroléine, dans lequel on guide un mélange de départ de gaz réactionnels contenant du propène, de l'oxygène moléculaire et au moins un gaz inerte, qui est constitué pour au moins 20% de son volume d'azote moléculaire, mélange qui contient l'oxygène moléculaire et le propène dans un rapport molaire de $O_2/C_3H_6 \geq 1$, à température élevée par un catalyseur à lit fixe, dont la masse active est au moins un oxyde multimétallique contenant du molybdène et/ou du tungstène ainsi que du bismuth, du tellure, de l'antimoine, de l'étain et/ou du cuivre, de façon que la conversion de propène soit, pour un passage unique, $\geq$ 90 moles % et la sélectivité ainsi développée de la formation d'acroléine ainsi que de la formation du produit secondaire acide acrylique soient conjointement $\geq$ 90 moles %, **caractérisé en ce que**

a) la charge du catalyseur à lit fixe par le propène contenu dans le mélange de départ de gaz réactionnels est $\geq$ 160 Nl de propène/l de masse de catalyseur·h,
b) le catalyseur à lit fixe est constitué d'une masse de catalyseur agencée en deux zones réactionnelles A, B spatialement successives, la température de la zone réactionnelle A étant de 300 à 390°C et la température de la zone réactionnelle B de 305 à 420°C et simultanément d'au moins 5°C supérieure à la température de la zone réactionnelle A,
c) le mélange de départ de gaz réactionnels traverse les zones réactionnelles A, B dans la succession dans le temps de "tout d'abord A", "ensuite B", et
d) la zone réactionnelle A s'étend jusqu'à une conversion du propène de 40 à 80 moles %.

**2.** Procédé suivant la revendication 1, **caractérisé en ce que** la zone réactionnelle A s'étend jusqu'à une conversion du propène de 50 à 70 moles %.

**3.** Procédé suivant la revendication 1, **caractérisé en ce que** la zone réactionnelle A s'étend jusqu'à une conversion du propène de 65 à 75 moles %.

**4.** Procédé suivant l'une des revendications 1 à 3, **caractérisé en ce que** la température de la zone réactionnelle B est d'au moins 10°C supérieure à la température de la zone réactionnelle A.

**5.** Procédé suivant l'une des revendications 1 à 4, **caractérisé en ce que** la température de la zone réactionnelle B est de 305°C à 340°C.

**6.** Procédé suivant l'une des revendications 1 à 4, **caractérisé en ce que** la température de la zone réactionnelle B est de 310°C à 330°C.

**7.** Procédé suivant l'une des revendications 1 à 6, **caractérisé en ce que** la conversion de propène est, pour un passage unique, ≥ 94 moles %.

**8.** Procédé suivant l'une des revendications 1 à 7, **caractérisé en ce que** la sélectivité de la formation d'acroléine ainsi que de la formation du produit secondaire acide acrylique est conjointement ≥ 94 moles %.

**9.** Procédé suivant l'une des revendications 1 à 8, **caractérisé en ce que** la charge en propène de la masse de catalyseur est ≥ 165 Nl/l·h.

**10.** Procédé suivant l'une des revendications 1 à 8, **caractérisé en ce que** la charge en propène de la masse de catalyseur est ≥ 170 Nl/l·h.

**11.** Procédé suivant l'une des revendications 1 à 10, **caractérisé en ce que** le au moins un gaz inerte est constitué pour ≥ 40 % en volume d'azote moléculaire.

**12.** Procédé suivant l'une des revendications 1 à 10, **caractérisé en ce que** le au moins un gaz inerte est constitué pour ≥ 60% en volume d'azote moléculaire.

**13.** Procédé suivant l'une des revendications 1 à 12, **caractérisé en ce que** le au moins un gaz inerte comporte de la vapeur d'eau.

**14.** Procédé suivant l'une des revendications 1 à 13, **caractérisé en ce que** le au moins un gaz inerte comporte du $CO_2$ et/ou du CO.

**15.** Procédé suivant l'une des revendications 1 à 14, **caractérisé en ce qu'**il est effectué à une pression de travail de 0,5 à 3,5 bars.

**16.** Procédé suivant l'une des revendications 1 à 15, **caractérisé en ce que** le rapport molaire $O_2/C_3H_6$ dans le mélange de départ de gaz réactionnels est de 1,5 à 2,0.

**17.** Procédé suivant l'une des revendications 1 à 16, **caractérisé en ce que**, comme source d'oxygène, on utilise conjointement de l'air.

**18.** Procédé suivant l'une des revendications 1 à 17, **caractérisé en ce que** la teneur en propène du mélange de départ de gaz réactionnels est de 4 à 15% en volume.

**19.** Procédé suivant l'une des revendications 1 à 17, **caractérisé en ce que** la teneur en propène du mélange de départ de gaz réactionnels est de 5 à 12% en volume.

**20.** Procédé suivant l'une des revendications 1 à 17, **caractérisé en ce que** la teneur en propène du mélange de départ de gaz réactionnels est de 5 à 8% en volume.

**21.** Procédé suivant l'une des revendications 1 à 20, **caractérisé en ce que** la masse active du catalyseur à lit fixe est au moins un oxyde multimétallique de la formule générale I :

$$Mo_{12}Bi_aFe_bX^1_cX^2_dX^3_eX^4_fO_n \qquad (I)$$

dans laquelle les variables présentent la signification suivante :

$X^1 =$ nickel et/ou cobalt,
$X^2 =$ thallium, un métal alcalin et/ou un métal alcalinoterreux,
$X^3 =$ zinc, phosphore, arsenic, bore, antimoine, étain, cérium, plomb et/ou tungstène,
$X^4 =$ silicium, aluminium, titane et/ou zirconium,

a = 0,5 à 5,
b = 0,01 à 5,
c = 0 à 10,
d = 0 à 2,
e = 0 à 8,
f = 0 à 10, et
n = un nombre qui est déterminé par la valence et la fréquence des éléments différents de l'oxygène dans I.

**22.** Procédé suivant l'une des revendications 1 à 20, **caractérisé en ce que** la masse active du catalyseur à lit fixe est au moins un oxyde multimétallique de la formule générale II :

$$[Y1^2_{a'}Y^2_{b'}O_{x'}]_p[Y^3_{c'}Y^4_{d'}Y^5_{e'}Y^6_{f'}Y^7_{g'}Y^2_{h'}O^7_{y'}]_q \qquad (II),$$

dans laquelle les variables ont la signification suivante :

$Y^1$ = bismuth, tellure, antimoine, étain et/ou cuivre,
$Y^2$ = molybdène et/ou tungstène,
$Y^3$ = un métal alcalin, thallium et/ou samarium,
$Y^4$ = un métal alcalino-terreux, nickel, cobalt, cuivre, manganèse, zinc, étain, cadmium et/ou mercure,
$Y^5$ = fer, chrome, cérium et/ou vanadium,
$Y^6$ = phosphore, arsenic, bore et/ou antimoine,
$Y^7$ = un métal de terre rare, titane, zirconium, niobium, tantale, rhénium, ruthénium, rhodium, argent, or, aluminium, gallium, indium, silicium, germanium, plomb, thorium et/ou uranium, et
a' = 0,01 à 8,
b' = 0,1 à 30,
c' = 0 à 4,
d' = 0 à 20,
e' = 0 à 20,
f' = 0 à 6,
g' = 0 à 15,
h' = 8 à 16,
x', y' = des nombres, qui sont déterminés par la valence et la fréquence des éléments différents de l'oxygène dans II, et
p, q = des nombres, dont le rapport p/q est de 0,1 à 10,

contenant des zones de la composition chimique $Y^1_{a'}Y^2_{b'}O_{x'}$ qui sont étendues tridimensionnellement et délimitées de leur environnement local en raison de leur composition différente de leur environnement local et dont le plus grand diamètre est de 1 nm jusqu'à 100 μm.

**23.** Procédé suivant l'une des revendications 1 à 22, **caractérisé en ce que** la masse de catalyseur comporte des catalyseurs annulaires et/ou en forme de billes.

**24.** Procédé suivant la revendication 23, **caractérisé en ce que** la géométrie annulaire est la suivante:

| diamètre externe | 2 à 10 mm, |
|---|---|
| longueur | 2 à 10 mm, |
| épaisseur de paras | 1 à 3 mm. |

**25.** Procédé suivant la revendication 23, **caractérisé en ce que** le catalyseur en forme de billes est un catalyseur à coquille constitué d'un support en forme de bille (diamètre de 1 à 8 mm) et d'une coquille de masse active appliquée sur celui-ci (épaisseur de 10 à 1000 μm).

**26.** Procédé suivant l'une des revendications 1 à 25, **caractérisé en ce qu'**il est effectué dans un réacteur à faisceau tubulaire en deux zones.

**27.** Procédé suivant l'une des revendications 1 à 26, **caractérisé en ce que** la température de la zone réactionnelle A est de 300 à 350°C et la température de la zone réactionnelle B de 305 à 380°C.

28. Procédé suivant l'une des revendications 1 à 27, **caractérisé en ce que** la conversion de propène est, pour un passage unique, ≥ 92 moles %.

29. Procédé suivant l'une des revendications 1 à 28, **caractérisé en ce que** le catalyseur à lit fixe est une masse de catalyseur dont l'activité spécifique en volume augmente de manière continue, abrupte ou par gradins dans le sens d'écoulement du mélange réactionnel par dilution par de la matière inerte.

30. Procédé de préparation d'acide acrylique, **caractérisé en ce qu'**il comporte un procédé suivant l'une des revendications 1 à 30.